# EUROPEAN PATENT SPECIFICATION

(11) **EP 4 094 735 B1**
(45) Date of publication and mention of the grant of the patent: **20.08.2025**
(21) Application number: 22175031.8
(22) Date of filing: 24.05.2022
(51) Int. Cl.: A61F 13/15, A61F 13/538

(54) **METHOD FOR MAKING AN ABSORBING DEVICE AND ABSORBING DEVICE MADE WITH SAID METHOD.**
VERFAHREN ZUR HERSTELLUNG EINER ABSORBIERENDEN VORRICHTUNG UND NACH DIESEM VERFAHREN HERGESTELLTE ABSORBIERENDE VORRICHTUNG.
MÉTHODE DE FABRICATION D'UN DISPOSITIF D'ABSORPTION ET DISPOSITIF D'ABSORPTION FABRIQUÉ SELON LADITE MÉTHODE.

(30) Priority: 24.05.2021 IT 202100013340
(43) Date of publication of application: 30.11.2022
(73) Proprietor: Parmon S.p.A., 95032 Belpasso (CT) (IT)
(72) Inventor: FRONTERRÈ, Sergio, 95030 Sant'Agata li Battiati (CT) (IT); GUGLIELMINO, Alfio, 95039 Trecastagni (CT) (IT); AZZOLINA, Eleonora, 95125 Catania (IT)
(74) Representative: Bonini, Ercole

(56) References cited:
- EP-B1- 2 768 456
- KR-A- 20040 040 044
- US-A1- 2007 116 926
- US-A1- 2020 397 630
- US-B1- 6 528 439

## Description

The invention concerns a method for making an absorbing device suitable for both children and adults with characteristics of high liquid absorption and low liquid return to the surface in contact with the wearer's skin.

As is well known, absorbing devices, both for children and for women's and adults' hygiene in general, are particularly suitable for receiving biological liquids (urine and menstruation) that are released by the person and that pass through a specific absorption zone that is not in contact with the person's skin, so that discomfort due to liquid contact with the skin can be minimised.

Document US2020397630 describes an absorbent article which includes a topsheet formed of a section of formed nonwoven web material having an ordered arrangement of zones, each zone comprising one or more attenuated regions adjacent to one or more built-up regions, wherein the attenuated regions have a first average basis weight and the built-up regions have a second average basis weight, wherein the first average basis weight is less than the second average basis weight, the difference in basis weights corresponding to the disposition of the filaments according to the ordered arrangement.

The attenuated region(s) may include a plurality of discrete low bulk portions arranged in a pattern disposed along a longitudinal axis of the article, which facilitate the z-direction movement of fluid through the topsheet and inhibit spreading of a fluid stain along the topsheet in the x and y directions.

The prior art has developed different types of absorbing devices capable of retaining moisture mainly thanks to the use of polymers that are capable of absorbing a quantity of liquid even equal to 40/50 times the weight of the polymer itself.

For this purpose, according to the prior art, the absorbing pad of which the above-mentioned device is made up is composed of a mixture of a super absorbent polymer and cellulose fibres.

The super-absorbent polymer consists of granules and fine particles, generally derived from acrylic acid, selected from sodium acrylate, potassium acrylate and alkyl acrylate.

These polymer particles act like tiny sponges and are capable of retaining considerable amounts of liquid up to 40/50 times their weight.

Furthermore, when the polymer granules come into contact with aqueous liquids, they form a gel network that is not soluble in water.

The gels formed in this way do not have the ability to transport or disperse liquids, whereas this function is optimally performed by cellulose fibres.

These in fact transport the liquid and disperse it within the actually absorbing part of the absorbing device.

This absorbing part, more commonly referred to as the "absorbing pad", is positioned in the centre of the absorbing device and is inserted between two layers of non-woven fabric **TNT** in order to give the same pad stability and mechanical strength.

The absorbing pad is then covered with an additional layer of non-woven fabric TNT, which performs the function of acquiring liquids and comes into contact with the person's skin.

Between the outer layer of non-woven fabric TNT and the absorbing pad, positioned in the centre of the absorbing device, there is an additional layer of non-woven fabric TNT which performs the specific function of acquiring the liquids and transferring them to the absorbing pad.

The method relating to the invention concerns precisely the maximisation of the liquid acquisition function, in the sense of achieving a transfer of the liquid from this layer to the absorbing pad with maximum speed, so as to minimise the wet feeling that the person may perceive.

More particularly, as will be seen below, the method of implementing the absorbency of such a layer is achieved by using ultraviolet rays UV that geometrically transform the fibres of the layer concerned by imbibition.

Further features and specificities of the invention will be better illustrated in the following description, provided by way of non-limiting example, with the help of the attached figures where:
- Fig. 1 shows the absorbing device in section;
- Fig. 2 shows in schematic form the tunnel where the absorbing devices pass under a conveyor belt and are subjected to the treatment with ultraviolet rays UV;
- Fig. 3 shows a section of Fig. 2;
- Fig. 4 shows in a diagram the relationship between the power for the surface unit irradiated by ultraviolet rays UV versus the thickness of the liquid acquisition and distribution layer;
- Fig. 5 shows in section the fibres of the liquid acquisition and distribution layer after irradiation with ultraviolet rays UV;
- Fig. 6 shows in section the fibres of the layer of Fig. 5 that are ordered by the action of ultraviolet rays UV.

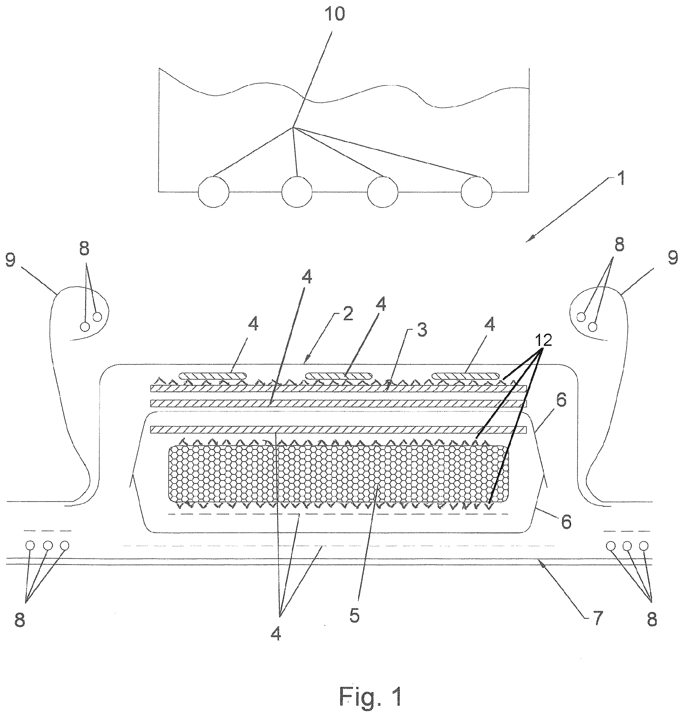

Referring now to Fig. 1, the absorbent device can be observed, indicated overall by **1,** in section and already fully formed.

In this absorbing device **1** there is a first layer of non-woven fabric **TNT** and polyethylene film, indicated with **2,** where this layer comes into contact with the person using the absorbing device **1,** whether child or adult.

The layer **2** is made adherent to the subsequent second layer **3** by means of a film of glue indicated with **4** that allows precisely the adhesion of the layer **2** in contact with the skin with the second lower layer **3** that is the layer of material that acquires and distributes the liquids to the third layer **5** that is the absorbing pad indicated with **5.**

The second layer **3** is a type of non-woven fabric TNT: in the case under examination, and by way of illustration but not limitation, use is made of the specific absorption/distribution fabric called ADL (Acquisition Distribution Layer) and which has precisely the function of acquiring the liquids that are lost by the person in order to distribute them and then to transfer them to the third layer **5** functioning as an absorbing pad.

This absorbing pad or third layer **5** generally consists of a SAP (Super Absorbent Polymer), generally hydrogel polymer, and cellulose fibres.

The absorbing pad **5** is also contained within a non-woven fabric TNT that wraps it so as to make it stable during absorption of the liquid and to prevent it from falling apart during use.

The absorbing pad or third layer **5** is also fixed through the film of glue **4** to the closing element **6,** which in turn adheres to the liquid acquisition and distribution layer **3.**

Then, there is a base layer **7,** also made of polyethylene, which serves as a support and containment of the absorbing device **1** when it is wrapped around the person.

There are also elastic elements, indicated with **8,** which are arranged in the barriers **9** of the absorbing device **1** that serve precisely to the same absorbing device **1** to remain in the position of use when worn by a person.

Again in Fig. 1, and in schematic form, four lamps indicated with **10** for the irradiation of the ultraviolet rays UV are indicated in section.

The sterilising power that ultraviolet rays UV exert on microorganisms and bacteria, which are pathogenic to human health, is well known and is used by various manufacturers.

What is not known, however, is a specific effect identified and obtained by the inventors, which allows ultraviolet rays UV, if arranged at a certain distance and if using a specific power striking the absorbing device **1,** to act in an effective and surprising manner on the second layer **3** of liquid acquisition and distribution by changing the nature of the distribution of the fibres, particularly by arranging them in a vertical direction, i.e. towards the source of irradiation, and consequently increasing the thickness of the second layer **3.**

As mentioned, the second layer **3** of liquid acquisition and distribution is formed by non-woven fabric TNT (usually ADL) of polyester fibre.

Now, under the action of a certain power irradiated by the ultraviolet rays UV, the polyester fibres of which the ADL layer is composed arrange themselves in a vertical direction and thus achieve the effect of sucking up liquids more quickly and transferring them just as quickly into the absorbing pad **5.**

To achieve this specific effect, experiments were carried out that took into account the fact that the absorbing devices, due to production necessity during their formation and their packaging, are arranged on a conveyor belt **11** moving at a relatively high speed.

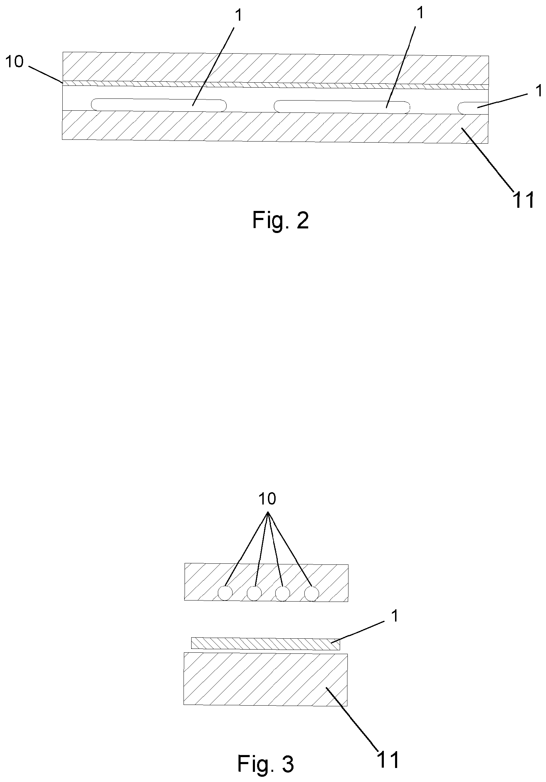

In this context, lamps **10** for the irradiation of ultraviolet rays UV were arranged along the direction of the conveyor belt **11,** as can be seen in Fig. 2, at a certain point in the packaging and before the absorbing device 1 is enclosed and thus in the process of being fully spread out.

As can be seen from Fig. 2 and Fig. 3, there are four lamps **10,** in the case of the example, with a length of about 90 cm and a power of about 41W. The distance between the lamps **10** and the conveyor belt **11** is about 20-25 mm.

In total, therefore, a supply power of 165W is developed with a total UVC power of 65W UVC.

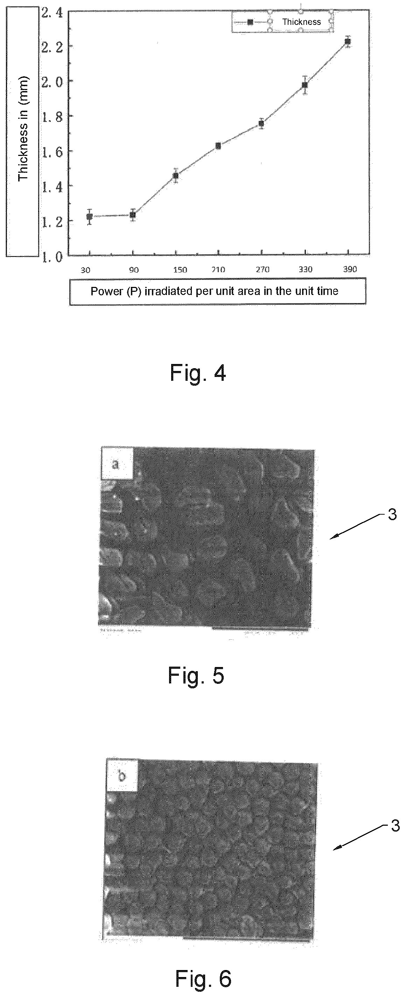

Experiments carried out by the inventors have shown that the variation in the thickness of the layer **3** exposed to ultraviolet rays UV is a function of the power, as is shown in the graph in Fig. 4, where increasing UV power increases the thickness of the second layer **3.**

Considering the speed of the line on which the absorbing device **1** rests, it results that 161 cm²/sec of fabric layer are subjected to irradiation in the unit of time.

Thus, the engaged and irradiated power per unit area in the unit time will be: 65W : 161 cm²/sec = 403,7 mWcm²/sec.

If we now look at Fig. 4, we see that this power per unit area causes an increase in thickness from 1 to 2.2/2.3 mm.

Now, Figs. 5 and 6 depict photographs taken by the inventors before and after irradiation with ultraviolet rays UV, respectively.

As can be seen, the fibres of the acquisition layer, after irradiation with ultraviolet rays UV, were arranged in an orderly and aligned manner.

In this way, the maximum distribution of liquids and their fastest transfer to the absorbing pad or third layer **5** is achieved.

According to a variant of the invention, given by way of non-limiting example, the absorbing device **1** described above has inside it microcapsules **12** of known type and containing essential oils within them which are intended to produce a soothing effect on the wearer of said device.

The microcapsules **12** in the example are shown in Fig. 1.

The capsules contain in their inside one or more essential oils such as Calendula Oil, Avocado Oil, Jojoba Oil, Olive Oil and Talcum Scent.

A greater soothing effect has been noted if the essential oils are present in the following percentages:
- Calendula Oil 59.8%
- Avocado Oil 20%
- Jojoba Oil 10%
- Olive Oil 10%
- Talcum Scent 0.2%.

These microcapsules, after being deposited on one or more layers of non-woven fabric (TNT) by means of a simple powder dispenser, are permanently fixed to the TNT support by the action of the ultraviolet rays UV acting on the absorbing device **1.**

Of course, in another embodiment example, the microcapsules **12** may be distributed only on the layer **3** or on the layer **5.**

The main purpose of the invention was thus achieved by creating an absorbing device **1** that increases the comfort of the wearer while minimising the wet feeling.

All this without modifying the formation of the device, but significantly increasing its absorption capacity and pleasantness.

## Claims

1. Method for implementing the absorbency and the distribution of liquids in an absorbing device (1), said absorbing device (1) comprising at least:
- a first upper layer (2) of fibre consisting of non-woven fabric (TNT) in contact with the user's skin;
- a second layer (3) of fibre consisting of non-woven fabric (TNT), in contact with said first layer (2), adapted to acquire the liquids received from said first layer (2) and to distribute said liquids to a third layer below;
- a third layer (5) below adapted to capture and absorb the liquids received from said second layer (3),
**characterised by** subjecting said absorbing device (1) to an irradiation by ultraviolet rays (UV), such as to modify the distribution of the fibres of said second layer (3) by ordering them along a direction essentially perpendicular to the surface of the absorbing device (1) spread out and increasing the thickness of said second layer (3),
- said ultraviolet rays (UV) transmitting to said absorbing device (1) a power of not less than 14.8 mWsec/cm²;
- lamps (10) of said ultraviolet rays UV being arranged according to the length of the conveyor belt (11) of said absorbing devices (1) at a distance not exceeding 20-25 mm.

2. Method according to claim 1, **characterised in that** said ultraviolet rays (UV) have an intensity of around 403 mWsec/cm².

3. Method according to any one of claims 1 to 2, **characterised in that** said second layer (3) of fibre of non-woven fabric (TNT) is of the ADL type (Acquisition Distribution Layer).

4. Absorbing device (1) subjected to the irradiation of the ultraviolet rays (UV) as defined in claim 1, **characterised in that** said third layer (5) comprises a mixture of polyacrylic sodium and cellulose fibres, the fibres of said second layer (3) being distributed and ordered along a direction essentially perpendicular to the surface of the absorbing device (1), resulting from the irradiation with UV.

5. Absorbing device (1) according to claim 4), **characterised in that** in one or more layers of the non-woven fabric (TNT) there are microcapsules (12) containing essential oils with a soothing effect, said microcapsules (12) being fixed to the non-woven fabric (TNT) by the action of ultraviolet rays (UV).

6. Absorbing device (1) according to claim 5), **characterised in that** the essential oils present in these microcapsules (12) are Calendula Oil, Avocado Oil, Jojoba Oil, Olive Oil and Talcum Scent.

7. Absorbing device (1) according to claim 6), **characterised in that** said essential oils are contained in the following percentages:
- Calendula Oil 59.8%;
- Avocado Oil 20%;
- Jojoba Oil 10%;
- Olive Oil 10%;
- Talcum Scent 0.2%.

## Patentansprüche

1. Verfahren zum Implementieren des Absorptionsvermögens und der Verteilung von Flüssigkeiten in einer Absorptionsvorrichtung (1), wobei die besagte Absorptionsvorrichtung (1) mindestens Folgendes umfasst:
- eine erste obere Faserschicht (2) aus Vliesstoff (TNT), die in Kontakt mit der Haut des Benutzers kommt;
- eine zweite Faserschicht (3) aus Vliesstoff (TNT), die in Kontakt mit der besagten ersten Schicht (2) kommt und dazu geeignet ist, die von der besagten ersten Schicht (2) erhaltenen Flüssigkeiten aufzunehmen und die besagten Flüssigkeiten auf eine darunter liegende dritte Schicht zu verteilen;
- eine darunter liegende dritte Schicht (5), die dazu geeignet ist, die von der besagten zweiten Schicht (3) erhaltenen Flüssigkeiten zurückzuhalten und zu absorbieren,
**dadurch gekennzeichnet, dass** die besagte Absorptionsvorrichtung (1) einer Bestrahlung mit ultravioletten Strahlen (UV) unterzogen wird, um die Verteilung der Fasern der besagten zweiten Schicht (3) zu ändern, indem sie entlang einer Richtung im Wesentlichen senkrecht zur Oberfläche der ausgebreiteten Absorptionsvorrichtung (1) geordnet werden und die Dicke der besagten zweiten Schicht (3) vergrößert wird,
- wobei die besagten ultravioletten Strahlen (UV) eine Leistung von nicht weniger als 14,8 mWs/cm² an die besagte Absorptionsvorrichtung (1) übertragen;
- wobei Lampen (10) der besagten ultravioletten Strahlen UV entsprechend der Länge des Förderbands (11) der besagten Absorptionsvorrichtungen (1) in einem Abstand von nicht mehr als 20-25 mm angeordnet sind.

2. Verfahren nach Patentanspruch 1, **dadurch gekennzeichnet, dass** die besagten ultravioletten Strahlen (UV) eine Intensität von etwa 403 mWs/cm² aufweisen.

3. Verfahren nach jeglichem der Patentansprüche 1 bis 2, **dadurch gekennzeichnet, dass** die besagte zweite Faserschicht (3) aus Vliesstoff (TNT) vom ADL-Typ (Acquisition Distribution Layer) ist.

4. Absorptionsvorrichtung (1), die der Bestrahlung mit ultravioletten Strahlen (UV), wie in Patentanspruch 1 definiert, unterzogen wird, **dadurch gekennzeichnet, dass** die besagte dritte Schicht (5) eine Mischung aus Polyacrylnatrium und Cellulosefasern umfasst, wobei die Fasern der besagten zweiten Schicht (3), als Resultat der Bestrahlung mit UV, entlang einer Richtung im Wesentlichen senkrecht zur Oberfläche der Absorptionsvorrichtung (1) verteilt und geordnet sind.

5. Absorptionsvorrichtung (1) nach Patentanspruch 4, **dadurch gekennzeichnet, dass** in einer oder mehreren Schichten des Vliesstoffs (TNT) Mikrokapseln (12) vorhanden sind, die ätherische Öle mit beruhigender Wirkung enthalten, wobei die besagten Mikrokapseln (12) durch Einwirkung von ultravioletten Strahlen (UV) am Vliesstoff (TNT) fixiert sind.

6. Absorptionsvorrichtung (1) nach Patentanspruch 5, **dadurch gekennzeichnet, dass** die ätherischen Öle in diesen Mikrokapseln (12) Ringelblumenöl, Avocadoöl, Jojobaöl, Olivenöl und Talkumduft sind.

7. Absorptionsvorrichtung (1) nach Patentanspruch 6, **dadurch gekennzeichnet, dass** die besagten ätherischen Öle in den folgenden Prozentsätzen enthalten sind:
- Ringelblumenöl 59,8%;
- Avocadoöl 20%;
- Jojobaöl 10%;
- Olivenöl 10%;
- Talkumduft 0,2%.

## Revendications

1. Procédé de mise en œuvre de l'absorption et de la distribution de liquides dans un dispositif absorbant (1), ledit dispositif absorbant (1) comprenant au moins:
- une première couche supérieure (2) de fibres constituée d'un tissu non tissé (TNT) en contact avec la peau de l'utilisateur;
- une deuxième couche (3) de fibres constituée d'un tissu non tissé (TNT), en contact avec ladite première couche (2), adaptée pour absorber les liquides provenant de ladite première couche (2) et pour distribuer lesdits liquides à une troisième couche située en dessous;
- une troisième couche (5) située en dessous adaptée pour recueillir et absorber les liquides provenant de ladite deuxième couche (3),
**caractérisé en ce que** l'on soumet ledit dispositif absorbant (1) à une irradiation par des rayons ultraviolets (UV), de manière à modifier la distribution des fibres de ladite deuxième couche (3) en les ordonnant selon une direction essentiellement perpendiculaire à la surface du dispositif absorbant (1) étalé et en augmentant l'épaisseur de ladite deuxième couche (3),
- lesdits rayons ultraviolets (UV) transmettant audit dispositif absorbant (1) une puissance d'au moins 14,8 mWsec/cm²;
- des lampes (10) desdits rayons ultraviolets UV étant disposées selon la longueur de la bande transporteuse (11) desdits dispositifs absorbants (1) à une distance ne dépassant pas 20-25 mm.

2. Procédé selon la revendication 1, **caractérisé en ce que** lesdits rayons ultraviolets (UV) ont une intensité d'environ 403 mWsec/cm².

3. Procédé selon l'une quelconque des revendications 1 à 2, **caractérisé en ce que** ladite deuxième couche (3) de fibres de tissu non tissé (TNT) est du type ADL (Acquisition Distribution Layer).

4. Dispositif absorbant (1) soumis à l'irradiation des rayons ultraviolets (UV) tel que défini dans la revendication 1, **caractérisé en ce que** ladite troisième couche (5) comprend un mélange de fibres de polyacrylate de sodium et de cellulose, les fibres de ladite deuxième couche (3) étant distribuées et ordonnées selon une direction essentiellement perpendiculaire à la surface du dispositif absorbant (1), résultant de l'irradiation aux UV.

5. Dispositif absorbant (1) selon la revendication 4, **caractérisé en ce que** dans une ou plusieurs couches du tissu non tissé (TNT) se trouvent des microcapsules (12) contenant des huiles essentielles à effet apaisant, lesdites microcapsules (12) étant fixées au tissu non tissé (TNT) par l'action des rayons ultraviolets (UV).

6. Dispositif absorbant (1) selon la revendication 5, **caractérisé en ce que** les huiles essentielles présentes dans ces microcapsules (12) sont l'huile de calendula, l'huile d'avocat, l'huile de jojoba, l'huile d'olive et le parfum de talc.

7. Dispositif absorbant (1) selon la revendication 6, **caractérisé en ce que** lesdites huiles essentielles sont contenues dans les pourcentages suivants:
- Huile de calendula 59,8%;
- Huile d'avocat 20%;
- Huile de jojoba 10%;
- Huile d'olive 10%;
- Parfum de talc 0,2%.
